(19) Europäisches Patentamt
European Patent Office
Office européen des brevets

(11) Veröffentlichungsnummer: **0 558 999 A2**

# (12) EUROPÄISCHE PATENTANMELDUNG

(21) Anmeldenummer: **93102464.0**

(22) Anmeldetag: **17.02.93**

(51) Int. Cl.[5]: **C07D 231/56**, C07D 231/12, C07D 231/16, C07D 231/54, A01N 43/56

(30) Priorität: **02.03.92 DE 4206531**

(43) Veröffentlichungstag der Anmeldung: **08.09.93 Patentblatt 93/36**

(84) Benannte Vertragsstaaten: **BE CH DE FR GB IT LI NL**

(71) Anmelder: **BAYER AG**

**D-51368 Leverkusen(DE)**

(72) Erfinder: **Schallner, Otto, Dr.**
**Noldeweg 22**
**W-4019 Monheim(DE)**
Erfinder: **Tiemann, Ralf, Dr.**
**Ernst-Ludwig-Kirchner-Strasse 5**
**W-5090 Leverkusen 1(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch(DE)**
Erfinder: **Vosswinkel, Renate, Dr.**
**Sankt-Maternus-Eck 14a**
**W-5067 Kürten-Bechen(DE)**

## (54) N-Aryl-Pyrazolderivative als Herbizide.

(57) Die Erfindung betrifft neue N-Aryl-Stickstoffheterocyclen der allgemeinen Formel (I)

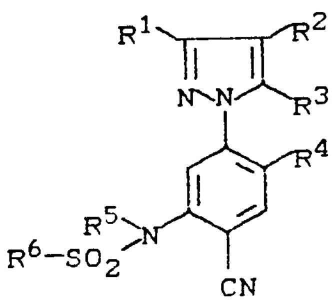

(I)

in welcher

| | |
|---|---|
| $R^1$ | für Wasserstoff oder einen Rest der Reihe Alkyl oder Halogenalkyl steht und |
| $R^2$ | für Wasserstoff, Halogen, Nitro oder einen Rest der Reihe Alkyl oder Halogenalkyl steht oder |
| $R^1$ und $R^2$ | gemeinsam für einen zweifach verknüpften Alkandiylrest stehen, |
| $R^3$ | für Wasserstoff, Halogen oder einen Rest der Reihe Alkyl oder Halogenalkyl steht, |
| $R^4$ | für Wasserstoff oder Halogen steht, |
| $R^5$ | für Wasserstoff oder Alkyl steht und |
| $R^6$ | für einen jeweils gegebenenfalls substituierten Rest der Reihe Alkyl, Alkenyl, Alkinyl, Cycloalkyl oder Aryl steht, |

ein Verfahren zu ihrer Herstellung und ihre Verwendung als Herbizide.

EP 0 558 999 A2

Die Erfindung betrifft neue N-Aryl-Stickstoffheterocyclen, ein Verfahren zu ihrer Herstellung und ihre Verwendung als Herbizide.

Es ist bekannt, daß bestimmte N-Aryl-Stickstoffheterocyclen, wie beispielsweise die Verbindung 4-Chlor-3,5-dimethyl-1-(4-cyano-2-fluor-5-methoxycarbonylmethylthiophenyl)-pyrazol herbizide Eigenschaften besitzen (vergleiche z.B. DE 38 39 480).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue N-Aryl-Stickstoffheterocyclen der allgemeinen Formel (I),

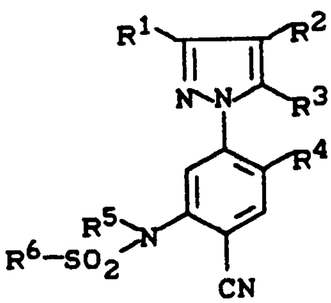

(I)

in welcher

| | |
|---|---|
| $R^1$ | für Wasserstoff oder einen Rest der Reihe Alkyl oder Halogenalkyl steht und |
| $R^2$ | für Wasserstoff, Halogen, Nitro oder einen Rest der Reihe Alkyl oder Halogenalkyl steht oder |
| $R^1$ und $R^2$ | gemeinsam für einen zweifach verknüpften Alkandiylrest stehen, |
| $R^3$ | für Wasserstoff, Halogen oder einen Rest der Reihe Alkyl oder Halogenalkyl steht, |
| $R^4$ | für Wasserstoff oder Halogen steht, |
| $R^5$ | für Wasserstoff oder Alkyl steht und |
| $R^6$ | für einen jeweils gegebenenfalls substituierten Rest der Reihe Alkyl, Alkenyl, Alkinyl, Cycloalkyl oder Aryl steht, |

gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen N-Aryl-Stickstoffheterocyclen der allgemeinen Formel (I),

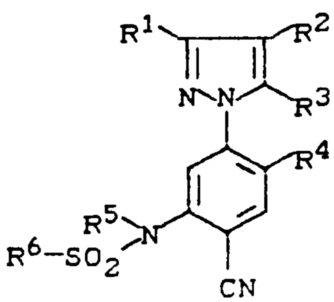

(I)

in welcher

| | |
|---|---|
| $R^1$ | für Wasserstoff oder einen Rest der Reihe Alkyl oder Halogenalkyl steht und |
| $R^2$ | für Wasserstoff, Halogen, Nitro oder einen Rest der Reihe Alkyl oder Halogenalkyl steht oder |
| $R^1$ und $R^2$ | gemeinsam für einen zweifach verknüpften Alkandiylrest stehen, |

$R^3$ für Wasserstoff, Halogen oder einen Rest der Reihe Alkyl oder Halogenalkyl steht,
$R^4$ für Wasserstoff oder Halogen steht,
$R^5$ für Wasserstoff oder Alkyl steht und
$R^6$ für einen jeweils gegebenenfalls substituierten Rest der Reihe Alkyl, Alkenyl, Alkinyl, Cycloalkyl oder Aryl steht,

erhält, wenn man
Fluorphenyl-Heterocyclen der Formel (II),

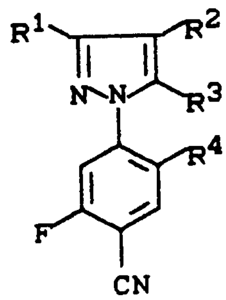

(II)

in welcher
$R^1$, $R^2$, $R^3$ und $R^4$ die oben angegebenen Bedeutungen haben,
mit Sulfonamiden der Formel (III),

$R^6$-$SO_2$-NH-$R^5$ (III)

in welcher
$R^5$ und $R^6$ die oben angegebenen Bedeutungen haben,
gegebenenfalls in Gegenwart eines Verdünnungsmittels und gegebenenfalls in Gegenwart eines Reaktionshilfsmittels umsetzt.

Schließlich wurde gefunden, daß die neuen N-Aryl-Stickstoffheterocyclen der allgemeinen Formel (I) herbizide Eigenschaften besitzen.

Überraschenderweise zeigen die erfindungsgemäßen N-Aryl-Stickstoffheterocyclender allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen, im Vergleich zu den aus dem Stand der Technik bekannten N-Aryl-Stickstoffheterocyclen, wie beispielsweise die Verbindung 4-Chlor-3,5-dimethyl-1-(4-cyano-2-fluor-5-methoxycarbonylmethylthio-phenyl)-pyrazol, welche chemisch und wirkungsmäßig naheliegende Verbindungen sind.

Die erfindungsgemäßen N-Arylheterocyclen sind durch die Formel (I) allgemein definiert. Bevorzugt sind Verbindungen der Formel (I), bei welchen

$R^1$ für Wasserstoff, für einen geradkettigen oder verzweigten Rest der Reihe Alkyl mit 1 bis 6 Kohlenstoffatomen oder Halogenalkyl mit 1 bis 6 Kohlenstoffatomen und 1 bis 13 gleichen oder verschiedenen Halogenatomen steht und
$R^2$ für Wasserstoff, Fluor, Chlor, Brom, Iod, Nitro, für einen geradkettigen oder verzweigten Rest der Reihe Alkyl mit 1 bis 6 Kohlenstoffatomen oder Halogenalkyl mit 1 bis 6 Kohlenstoffatomen und 1 bis 13 gleichen oder verschiedenen Halogenatomen steht oder
$R^1$ und $R^2$ gemeinsam für einen geradkettigen oder verzweigten zweifach verknüpften Alkandiylrest mit 2 bis 6 Kohlenstoffatomen stehen,
$R^3$ für Wasserstoff, Fluor, Chlor, Brom, Iod, für einen geradkettigen oder verzweigten Rest der Reihe Alkyl mit 1 bis 6 Kohlenstoffatomen oder Halogenalkyl mit 1 bis 6 Kohlenstoffatomen und 1 bis 13 gleichen oder verschiedenen Halogenatomen steht,
$R^4$ für Wasserstoff, Fluor, Chlor, Brom, oder Iod steht,
$R^5$ für Wasserstoff oder für geradkettiges oder verzweigtes Alkyl mit 1 bis 6 Kohlenstoffatomen steht und
$R^6$ für einen geradkettigen oder verzweigten
Rest der Reihe Alkyl mit
1 bis 18 Kohlenstoffatomen, Alkenyl oder Alkinyl mit jeweils 2 bis 8 Kohlenstoffatomen, Halogenalkyl mit 1 bis 12 Kohlenstoffatomen und 1 bis 25 gleichen oder verschiedenen

Halogenatomen, Cycloalkyl mit 3 bis 8 Kohlenstoffatomen oder für gegebenenfalls einfach oder mehrfach, gleich oder verschieden substituiertes Aryl mit 6 bis 10 Kohlenstoffatomen steht, wobei als Sustituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl.

Besonders bevorzugt sind Verbindungen der Formel (I), bei welchen

$R^1$ für Wasserstoff, für einen geradkettigen oder verzweigten Rest der Reihe Alkyl mit 1 bis 4 Kohlenstoffatomen oder Halogenalkyl mit 1 bis 4 Kohlenstoffatomen und 1 bis 9 gleichen oder verschiedenen Halogenatomen steht und

$R^2$ für Wasserstoff, Fluor, Chlor, Brom, Nitro, für einen geradkettigen oder verzweigten Rest der Reihe Alkyl mit 1 bis 4 Kohlenstoffatomen oder Halogenalkyl mit 1 bis 4 Kohlenstoffatomen und 1 bis 9 gleichen oder verschiedenen Halogenatomen steht oder

$R^1$ und $R^2$ gemeinsam für einen geradkettigen oder verzweigten zweifach verknüpften Alkandiylrest mit 3 bis 6 Kohlenstoffatomen stehen,

$R^3$ für Wasserstoff, Fluor, Chlor, Brom, für einen geradkettigen oder verzweigten Rest der Reihe Alkyl mit 1 bis 4 Kohlenstoffatomen oder Halogenalkyl mit 1 bis 4 Kohlenstoffatomen und 1 bis 9 gleichen oder verschiedenen Halogenatomen steht,

$R^4$ für Wasserstoff, Fluor, Chlor oder Brom steht,

$R^5$ für Wasserstoff oder für geradkettiges oder verzweigtes Alkyl mit 1 bis 4 Kohlenstoffatomen steht und

$R^6$ für einen geradkettigen oder verzweigten
Rest der Reihe Alkyl mit
1 bis 12 Kohlenstoffatomen, Alkenyl oder Alkinyl mit jeweils 2 bis 6 Kohlenstoffatomen, Halogenalkyl mit 1 bis 10 Kohlenstoffatomen und 1 bis 21 gleiche oder verschiedenen Halogenatomen, Cycloalkyl mit 3 bis 7 Kohlenstoffatomen oder für gegebenenfalls einfach bis fünffach, gleich oder verschieden substituiertes Aryl mit 6 oder 10 Kohlenstoffatomen steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl.

Ganz besonders bevorzugt sind Verbindungen der Formel (I), bei welchen

$R^1$ für Wasserstoff, für Methyl, Ethyl, n- oder i-Propyl oder für geradkettiges oder verzweigtes Halogenalkyl mit 1 bis 3 Kohlenstoffatomen und 1 bis 7 gleichen oder verschiedenen Halogenatomen - insbesondere Fluor, Chlor oder Brom - steht und

$R^2$ für Wasserstoff, Chlor, Brom, Nitro, für Methyl, Ethyl, n- oder i-Propyl oder für geradkettiges oder verzweigtes Halogenalkyl mit 1 bis 3 Kohlenstoffatomen und 1 bis 7 gleichen oder verschiedenen Halogenatomen - insbesondere Fluor, Chlor oder Brom - steht oder

$R^1$ und $R^2$ gemeinsam für einen geradkettigen oder verzweigten zweifach verknüpften Alkandiylrest mit 3 bis 4 Kohlenstoffatomen stehen,

$R^3$ für Wasserstoff, Fluor, Chlor, Brom, für Methyl, Ethyl, n- oder i-Propyl oder für geradkettiges oder verzweigtes Halogenalkyl mit 1 oder 2 Kohlenstoffatomen und 1 bis 5 gleichen oder verschiedenen Halogenatomen - insbesondere Fluor, Chlor oder Brom - steht,

$R^4$ für Wasserstoff, Fluor oder Chlor steht,

$R^5$ für Wasserstoff, Methyl, Ethyl, n- oder i-Propyl, n-, i-, s- oder t-Butyl steht und

$R^6$ für einen geradkettigen oder verzweigten Rest der Reihe Alkyl mit 1 bis 8 Kohlenstoffatomen, Halogenalkyl mit 1 bis 8 Kohlenstoffatomen und 1 bis 17 gleichen oder verschiedenen Halogenatomen - insbesondere Fluor, Chlor oder Brom - steht, außerdem für Cyclopropyl oder Cyclohexyl oder für gegebenenfalls einfach bis dreifach, gleich oder verschieden substituiertes Phenyl steht, wobei als Substituenten infrage kommen:

Fluor, Chlor, Brom, Cyano, Nitro, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl subtituiertes Phenyl.

Verwendet man beispielsweise 1-(4-Cyano-3-fluor-phenyl)-3,5-dimethyl-4-chlorpyrazol und Ethansulfonamid als Ausgangsstoffe, so läßt sich der Reaktionsablauf des erfindungsgemäßen Verfahrens durch das folgende Formelschema darstellen:

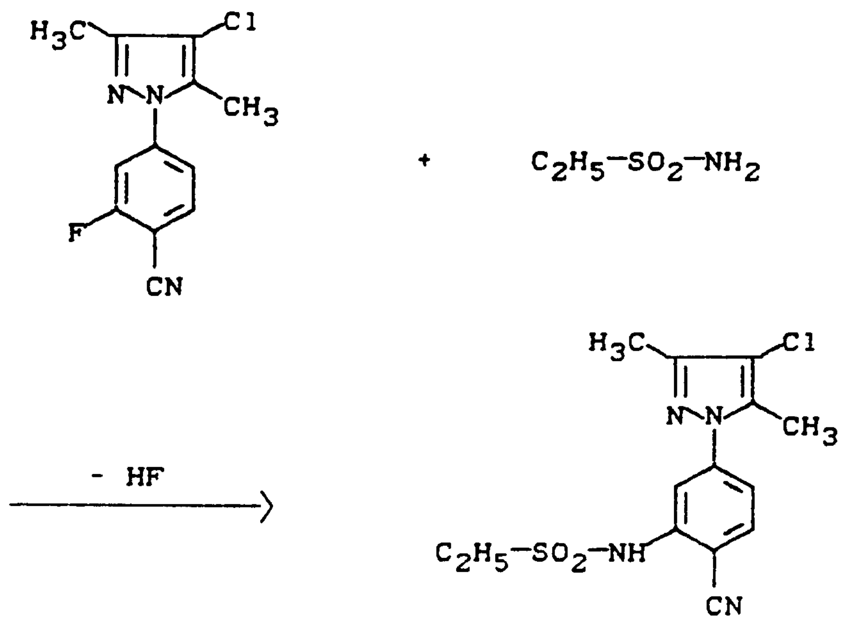


Die zur Durchführung des erfindungsgemäßen Verfahrens als Ausgangsstoffe benötigten Fluorphenyl-Heterocyclen sind durch die Formel (II) allgemein definiert. In dieser Formel (II) stehen $R^1$, $R^2$, $R^3$ und $R^4$ vorzugsweise für diejenigen Reste, die bereits im Zusammenhang mit der Beschreibung der erfindungsgemäßen Verbindungen der Formel (I) als bevorzugt für diesen Substituenten genannt wurden.
Die Fluorphenyl-Heterocyclen der Formel (II) sind bekannt oder erhältlich in Analogie zu bekannten Verfahren (vergleiche z.B. DE 39 03 799 oder DE 38 39 480).

Die zur Durchführung des erfindungsgemäßen Verfahrens weiterhin als Ausgangsstoffe benötigten Sulfonamide sind durch die Formel (III) allgemein definiert. In dieser Formel (III) stehen $R^5$ und $R^6$ vorzugsweise für diejenigen Reste, die bereits im Zusammenhang mit der Beschreibung der erfindungsgemäßen Verbindungen der Formel (I) als bevorzugt für diese Substituenten genannt wurden. Die Sulfonamide der Formel (III) sind allgemein bekannte Verbindungen der organischen Chemie.

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton, Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder Sulfoxide wie Dimethylsulfoxid.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate wie beispielsweise Natriumhydrid, Natriumamid, Natriummethylat, Natriumethylat, Kaliumtert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicylononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 20 °C und 180 °C, vorzugsweise bei Temperaturen zwischen 40 °C und 160 °C.

Das erfindungsgemäße Verfahren wird üblicherweise unter Normaldruck oder unter vermindertem Druck durchgeführt. Es ist jedoch auch möglich, unter erhöhtem oder vermindertem Durck zu arbeiten.

Zur Durchführung des erfindungsgemäßen Verfahrens setzt man pro Mol Fluorphenyl-Heterocyclus der Formel (II) im allgemeinen 0,8 bis 1,5 Mol, vorzugsweise 1,0 bis 1,2 Mol Sulfonamid der Formel (III) und gegebenenfalls 1,0 bis 3,0 Mol, vorzugsweise 1,0 bis 2,0 Mol Base als Reaktionshilfsmittel ein. Die Reaktionsdurchführung, Aufarbeitung und Isolierung der Reaktionsprodukte erfolgt nach bekannten Verfahren (vergleiche hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.
Die Charakterisierung erfolgt mit Hilfe des Schmelzpunktes oder bei nicht kristallisierenden Verbindungen mit Hilfe der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in monokotylen und dikotylen Kulturen wie beispielsweise Weizen, Mais, Raps oder Soja einsetzen.

Daneben greifen die erfindungsgemäßen Wirkstoffe auch in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen von dem Zeitpunkt der Anwendung bezogen auf das Entwicklungsstadium der Pflanze ab, sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch

vorgenommen werden, um die Transpiration der Pflanzen vor dem Verpflanzen herabzusetzen.

Darüberhinaus zeigen die erfindungsgemäßen Wirkstoffe in entsprechenden Aufwandmengen auch eine fungizide Wirksamkeit und können zur Bekämpfung von Reiskrankheiten, wie beispielsweise gegen den Erreger der Reisfleckenkrankheit (Pyricularia oryzae) eingesetzt werden. Daneben zeigen die erfindungsgemäßen Wirkstoffe auch eine gute in vitro-Wirksamkeit.

Die Wirkstoffe können in die üblichen Formulierungen überführt werden, wie Lösungen, Emulsionen, Suspensionen, Pulver, Schäume, Pasten, Granulate, Aerosole, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alko hole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate. Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die Herstellung und die Verwendung der erfindungsgemäßen Wirkstoffe geht aus den nachfolgenden Beispielen hervor.

## Herstellungsbeispiele

### Beispiel 1

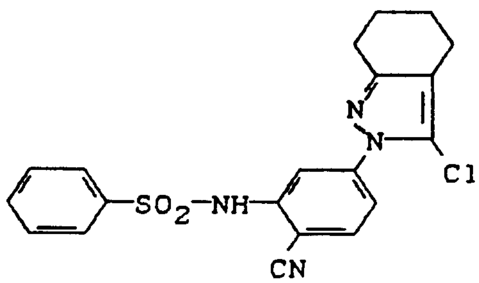

Zu einer Lösung von 4,1 g (0,015 Mol) 1-(4-Cyano-3-fluorphenyl)-5-chlor-3,4-tetramethylen-pyrazol (vergleiche z.B. DE 38 39 480) in 80 ml Dimethylsulfoxid gibt man nacheinander 2,3 g (0,016 Mol) Kaliumcarbonat und 2,4 g (0,015 Mol) Benzolsulfonamid und rührt 6 Stunden bei 135 °C bis 140 °C. Zur Aufarbeitung wird das Lösungsmittel im Vakuum abdestilliert, der Rückstand in Wasser aufgenommen, mit verdünnter Salzsäure auf pH 2 angesäuert und mit Dichlormethan extrahiert. Die vereinigten organischen Phasen werden über Magnesiumsulfat getrocknet und im Vakuum vom Lösungsmittel befreit.

Man erhält 6,1 g (98 % der Theorie) 1-(4-Cyano-3-benzolsulfonamido-phenyl)-5-chlor-3,4-tetramethylen-pyrazol vom Schmelzpunkt 177 °C.

In entsprechender Weise und gemäß den allgemeinen Angaben zur Herstellung erhält man die folgenden N-Arylheterocyclen der allgemeinen Formel (I):

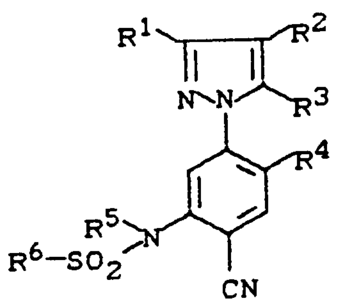

(I)

| Bsp.-Nr. | $R^1$ $R^2$ | $R^3$ | $R^4$ | $R^5$ | $R^6$ | Schmelzpunkt |
|---|---|---|---|---|---|---|
| 2 | $-(CH_2)_4-$ | Cl | H | H | $CH_3$ | Fp.177° C |
| 3 | $-(CH_2)_4-$ | Cl | F | H | $CH_3$ | Fp.181° C |
| 4 | $-(CH_2)_4-$ | $CH_3$ | F | H | $CH_3$ | Fp.198° C |
| 5 | $-(CH_2)_4-$ | Cl | F | H | $C_2H_5$ | Fp.168° C |
| 6 | $-(CH_2)_4-$ | Cl | F | H | $n-C_3H_7$ | Fp.57-61° C |
| 7 | $-(CH_2)_4-$ | Cl | H | H | $C_2H_5$ | Fp.156° C |
| 8 | $-(CH_2)_4-$ | Cl | H | H | $n-C_3H_7$ | Fp.140° C |
| 9 | $-(CH_2)_4-$ | Cl | H | H | $n-C_4H_9$ | Fp.94-96° C |

| Bsp.-Nr. | $R^1$ | $R^2$ | $R^3$ | $R^4$ | $R^5$ | $R^6$ | Schmelzpunkt |
|---|---|---|---|---|---|---|---|
| 10 | $-(CH_2)_4-$ | | Cl | F | H | $n-C_4H_9$ | Fp.65-71° C |
| 11 | $CH_3$ | $CH_3$ | $CH_3$ | F | H | $CH_3$ | Fp.>230° C |
| 12 | $CH_3$ | $CH_3$ | $CH_3$ | F | H | $n-C_4H_9$ | Fp.132° C |
| 13 | $-(CH_2)_4-$ | | Cl | F | H | $C_6H_5$ | Fp.208° C |
| 14 | $-(CH_2)_4-$ | | $CH_3$ | F | H | $n-C_4H_9$ | Fp.156° C |
| 15 | $CH_3$ | H | $CH_3$ | F | H | $CH_3$ | Fp.167° C |
| 16 | $CH_3$ | H | $CH_3$ | F | H | $n-C_4H_9$ | Fp.67° C |
| 17 | $CH_3$ | Cl | $CH_3$ | F | H | $CH_3$ | Fp.187° C |
| 18 | $CH_3$ | Cl | $CH_3$ | F | H | $n-C_4H_9$ | Fp. ° C |

| Bsp. Nr. | $R^1$ | $R^2$ | $R^3$ | $R^4$ | $R^5$ | $R^6$ | Schmelzpunkt |
|---|---|---|---|---|---|---|---|
| 19 | H | $NO_2$ | H | F | H | $CH_3$ | Fp. 181°C |
| 20 | H | $NO_2$ | H | F | H | n-$C_4H_9$ | Fp. 149°C |
| 21 | $CH_3$ | $NO_2$ | $CH_3$ | F | H | $CH_3$ | Fp. 178°C |
| 22 | $CH_3$ | $NO_2$ | $CH_3$ | F | H | n-$C_4H_9$ | Fp. 74°C |
| 23 | $CH_3$ | $CH_3$ | $CH_3$ | F | H | n-$C_4F_9$ | Fp. 234°C |
| 24 | $-(CH_2)_4-$ | | Cl | F | H | n-$C_8H_{17}$ | Fp. 83°C |
| 25 | $-(CH_2)_5-$ | | Cl | F | H | $CH_3$ | Fp. 67°C |
| 26 | $-(CH_2)_5-$ | | Cl | H | H | $CH_3$ | Fp. 163°C |
| 27 | $-(CH_2)_5-$ | | Cl | H | H | $C_2H_5$ | Fp. 119°C |
| 28 | $-(CH_2)_5-$ | | Cl | H | H | n-$C_4H_9$ | Fp. 76°C |
| 29 | $-(CH_2)_5-$ | | Cl | H | H | n-$C_8H_{17}$ | Öl |
| 30 | $-(CH_2)_4-$ | | Br | F | H | $CH_3$ | Fp. 199°C |
| 31 | $-(CH_2)_4-$ | | Br | F | H | n-$C_8H_{17}$ | Öl |
| 32 | $-(CH_2)_4-$ | | H | H | H | $CH_3$ | Fp. 187-188°C |
| 33 | $-(CH_2)_3-$ | | Cl | F | H | $CH_3$ | Öl |

Anwendungsbeispiele:

In den folgenden Anwendungsbeispielen wurde die nachstehend aufgeführte Verbindung als Vergleichssubstanz eingesetzt:

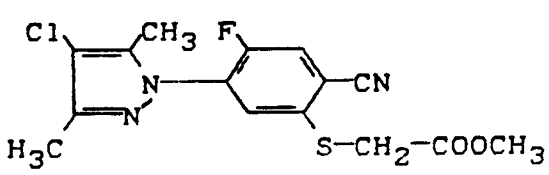

(A)

4-Chlor-3,5-dimethyl-1-(4-cyano-2-fluor-5-methoxycarbonylmethylthio-phenyl)-pyrazol
(bekannt aus DE 38 39 480)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstante. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigt in diesem Test z.B. die Verbindung gemäß Herstellungsbeispiel 3.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigen in diesem Test z.B. die Verbindungen gemäß folgender Herstellungsbeispiele: 2 und 4.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zur Entwicklung der unbehandelter Kontrolle bonitiert.

Eine deutliche Überlegenheit in der Wirksamkeit gegenürer dar unbehandelten Kontrolle zeigen in diesem Test z.B. die Verbindungen gemäß den Herstellungsbeispielen 3 und 4.

**Patentansprüche**

1. N-Aryl-Stickstoffheterocyclen der allgemeinen Formel (I)

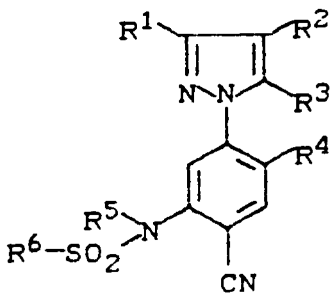

(I)

in welcher

| | |
|---|---|
| $R^1$ | für Wasserstoff oder einen Rest der Reihe Alkyl oder Halogenalkyl steht und |
| $R^2$ | für Wasserstoff, Halogen, Nitro oder einen Rest der Reihe Alkyl oder Halogenalkyl steht oder |
| $R^1$ und $R^2$ | gemeinsam für einen zweifach verknüpften Alkandiylrest stehen, |
| $R^3$ | für Wasserstoff, Halogen oder einen Rest der Reihe Alkyl oder Halogenalkyl steht, |
| $R^4$ | für Wasserstoff oder Halogen steht, |
| $R^5$ | für Wasserstoff oder Alkyl steht und |
| $R^6$ | für einen jeweils gegebenenfalls substituierten Rest der Reihe Alkyl, Alkenyl, Alkinyl, Cycloalkyl oder Aryl steht. |

2. N-Aryl-Stickstoffheterocyclen der allgemeinen Formel (I) gemäß Anspruch 1, dadurch gekennzeichnet, daß

| | |
|---|---|
| $R^1$ | für Wasserstoff, für einen geradkettigen oder verzweigten Rest der Reihe Alkyl mit 1 bis 6 Kohlenstoffatomen oder Halogenalkyl mit 1 bis 6 Kohlenstoffatomen und 1 bis 13 gleichen oder verschiedenen Halogenatomen steht und |
| $R^2$ | für Wasserstoff, Fluor, Chlor, Brom, Iod, Nitro, für einen geradkettigen oder verzweigten Rest der Reihe Alkyl mit 1 bis 6 Kohlenstoffatomen oder Halogenalkyl mit 1 bis 6 Kohlenstoffatomen und 1 bis 13 gleichen oder verschiedenen Halogenatomen steht oder |
| $R^1$ und $R^2$ | gemeinsam für einen geradkettigen oder verzweigten, zweifach verknüpften, Alkandiylrest mit 2 bis 6 Kohlenstoffatomen stehen, |
| $R^3$ | für Wasserstoff, Fluor, Chlor, Brom, Iod, für einen geradkettigen oder verzweigten Rest der Reihe Alkyl mit 1 bis 6 Kohlenstoffatomen oder Halogenalkyl mit 1 bis 6 Kohlenstoffatomen und 1 bis 13 gleichen oder verschiedenen Halogenatomen steht, |
| $R^4$ | für Wasserstoff, Fluor, Chlor, Brom, oder Iod steht, |
| $R^5$ | für Wasserstoff oder für geradkettiges oder verzweigtes Alkyl mit 1 bis 6 Kohlenstoffatomen steht und |
| $R^6$ | für einen geradkettigen oder verzweigten<br>Rest der Reihe<br>Alkyl mit 1 bis 18 Kohlenstoffatomen, Alkenyl oder Alkinyl mit jeweils 2 bis 8 Kohlenstoffatomen, Halogenalkyl mit 1 bis 12 Kohlenstoffatomen und 1 bis 25 gleichen oder verschiedenen Halogenatomen, Cycloalkyl mit 3 bis 8 Kohlenstoffatomen oder für gegebenenfalls einfach oder mehrfach, gleich oder verschieden substituiertes Aryl mit 6 bis 10 Kohlenstoffatomen steht, wobei als Sustituenten infrage kommen;<br>Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 |

bis 4 Kohlenstoffatomen substituiertes Phenyl.

**3.** N-Aryl-Stickstoffheterocyclen der allgemeinen Formel (I) gemäß Anspruch 1, dadurch gekennzeichnet, daß

$R^1$ für Wasserstoff, für einen geradkettigen oder verzweigten Rest der Reihe Alkyl mit 1 bis 4 Kohlenstoffatomen oder Halogenalkyl mit 1 bis 4 Kohlenstoffatomen und 1 bis 9 gleichen oder verschiedenen Halogenatomen steht und

$R^2$ für Wasserstoff, Fluor, Chlor, Brom, Nitro, für einen geradkettigen oder verzweigten Rest der Reihe Alkyl mit 1 bis 4 Kohlenstoffatomen oder Halogenalkyl mit 1 bis 4 Kohlenstoffato men und 1 bis 9 gleichen oder verschiedenen Halogenatomen steht oder

$R^1$ und $R^2$ gemeinsam für einen geradkettigen oder verzweigten zweifach verknüpften Alkandiylrest mit 3 bis 6 Kohlenstoffatomen stehen,

$R^3$ für Wasserstoff, Fluor, Chlor, Brom, für einen geradkettigen oder verzweigten Rest der Reihe Alkyl mit 1 bis 4 Kohlenstoffatomen oder Halogenalkyl mit 1 bis 4 Kohlenstoffatomen und 1 bis 9 gleichen oder verschiedenen Halogenatomen steht,

$R^4$ für Wasserstoff, Fluor, Chlor oder Brom steht,

$R^5$ für Wasserstoff oder für geradkettiges oder verzweigtes Alkyl mit 1 bis 4 Kohlenstoffatomen steht und

$R^6$ für einen geradkettigen oder verzweigten
Rest' der Reihe
Alkyl mit 1 bis 12 Kohlenstoffatomen, Alkenyl oder Alkinyl mit jeweils 2 bis 6 Kohlenstoffatomen, Halogenalkyl mit 1 bis 10 Kohlenstoffatomen und 1 bis 21 gleiche oder verschiedenen Halogenatomen, Cycloalkyl mit 3 bis 7 Kohlenstoffatomen oder für gegebenenfalls einfach bis fünffach, gleich oder verschieden substituiertes Aryl mit 6 oder 10 Kohlenstoffatomen steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl.

**4.** N-Aryl-Stickstoffheterocyclen der allgemeinen Formel (I) gemäß Anspruch 1, dadurch gekennzeichnet, daß

$R^1$ für Wasserstoff, für Methyl, Ethyl, n- oder i-Propyl oder für geradkettiges oder verzweigtes Halogenalkyl mit 1 bis 3 Kohlenstoffatomen und 1 bis 7 gleichen oder verschiedenen Halogenatomen - insbesondere Fluor, Chlor oder Brom - steht und

$R^2$ für Wasserstoff, Chlor, Brom, Nitro, für Methyl, Ethyl, n- oder i-Propyl oder für geradkettiges oder verzweigtes Halogenalkyl mit 1 bis 3 Kohlenstoffatomen und 1 bis 7 gleichen oder verschiedenen Halogenatomen - insbesondere Fluor, Chlor oder Brom -steht oder

$R^1$ und $R^2$ gemeinsam für einen geradkettigen oder verzweigten zweifach verknüpften Alkandiylrest mit 3 bis 4 Kohlenstoffatomen stehen,

$R^3$ für Wasserstoff, Fluor, Chlor, Brom, für Methyl, Ethyl, n- oder i-Propyl oder für geradkettiges oder verzweigtes Halogenalkyl mit 1 oder 2 Kohlenstoffatomen und 1 bis 5 gleichen oder verschiedenen Halogenatomen - insbesondere Fluor, Chlor oder Brom - steht,

$R^4$ für Wasserstoff, Fluor oder Chlor steht,

$R^5$ für Wasserstoff, Methyl, Ethyl, n- oder i-Propyl, n-, i-, s- oder t-Butyl steht und

$R^6$ für einen geradkettigen oder verzweigten Rest der Reihe Alkyl mit 1 bis 8 Kohlenstoffatomen, Halogenalkyl mit 1 bis 8 Kohlenstoffatomen und 1 bis 17 gleichen oder verschiedenen Halogenatomen - insbesondere Fluor, Chlor oder Brom - steht, außerdem für Cyclopropyl oder Cyclohexyl oder für gegebenenfalls einfach bis dreifach, gleich oder verschieden substituiertes Phenyl steht, wobei als Substituenten infrage kommen:
Fluor, Chlor, Brom, Cyano, Nitro, Ethyl, n-oder i-Propyl, n-, i-, s- oder t-Butyl, Methoxy, Ethoxy, n- oder i-Propoxy, n-, i-, s- oder t-Butoxy, Methylthio, Ethylthio,

Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl subtituiertes Phenyl.

**5.** Verfahren zur Herstellung von N-Aryl-Stickstoffheterocyclen der allgemeinen Formel (I)

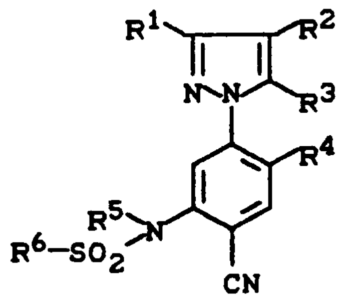

(I)

in welcher

| | |
|---|---|
| $R^1$ | für Wasserstoff oder einen Rest der Reihe Alkyl oder Halogenalkyl steht und |
| $R^2$ | für Wasserstoff, Halogen, Nitro oder einen Rest der Reihe Alkyl oder Halogenalkyl steht oder |
| $R^1$ und $R^2$ | gemeinsam für einen zweifach verknüpften Alkandiylrest stehen, |
| $R^3$ | für Wasserstoff, Halogen oder einen Rest der Reihe Alkyl oder Halogenalkyl steht, |
| $R^4$ | für Wasserstoff oder Halogen steht, |
| $R^5$ | für Wasserstoff oder Alkyl steht und |
| $R^6$ | für einen jeweils gegebenenfalls substituierten Rest der Reihe Alkyl, Alkenyl, Alkinyl, Cycloalkyl oder Aryl steht, |

dadurch gekenzeichnet, daß man
Fluorphenyl-Heterocyclen der Formel (II),

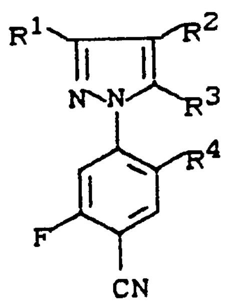

(II)

in welcher
$R^1$, $R^2$, $R^3$ und $R^4$ die oben angegebenen Bedeutungen haben,
mit Sulfonamiden der Formel (III),

$R^6$-$SO_2$-NH-$R^5$ (III)

in welcher
$R^5$ und $R^6$ die oben angegebenen Bedeutungen haben,
gegebenenfalls in Gegenwart eines Verdünnungsmittels und gegebenenfalls in Gegenwart eines Reaktionshilfsmittels umsetzt.

**6.** Herbizide Mittel, gekennzeichnet durch einen Gehalt an mindestens einem N-Aryl-Stickstoffheterocyclus der Formel (I) gemäß den Ansprüchen 1 bis 5.

7. Verfahren zur Bekämpfung von unerwünschten Pflanzen, dadurch gekennzeichnet, daß man N-Aryl-Stickstoffheterocyclen der Formel (I) gemäß den Ansprüchen 1 bis 5 auf unerwünschte Pflanzen und/oder ihren Lebensraum einwirken läßt.

8. Verwendung von N-Aryl-Stickstoffheterocyclen der Formel (I) gemäß den Ansprüchen 1 bis 5 zur Bekämpfung von unerwünschten Pflanzen.

9. Verfahren zur Herstellung von herbiziden Mitteln, dadurch gekennzeichnet, daß man N-Aryl-Stickstoffheterocyclen der Formel (I) gemäß den Ansprüchen 1 bis 5 mit Streckmitteln und/oder oberflächenaktiven Substanzen vermischt.